Europäisches Patentamt

European Patent Office

Office européen des brevets

(11) Publication number: **0 364 944 B1**

(12) **EUROPEAN PATENT SPECIFICATION**

(45) Date of publication of patent specification: **21.07.93** (51) Int. Cl.⁵: **A61K 9/18**

(21) Application number: **89119241.1**

(22) Date of filing: **17.10.89**

(54) **Poorly soluble medicaments supported on polymer substances in a form suitable for increasing their dissolving rate.**

(30) Priority: **17.10.88 IT 2233688**

(43) Date of publication of application:
**25.04.90 Bulletin 90/17**

(45) Publication of the grant of the patent:
**21.07.93 Bulletin 93/29**

(84) Designated Contracting States:
**AT BE CH DE ES FR GB GR IT LI LU NL SE**

(56) References cited:
**EP-A- 0 232 155**
**DE-A- 3 326 167**

**DIE PHARMAZIE, vol. 39, no. 7, July 1984, pages 473-475, East-Berlin, DD; E. FENYVEST et al.: "Cyclodextrin polymer, a new tablet disintegrating agent"**

(73) Proprietor: **VECTORPHARMA INTERNATIONAL S.P.A.**
**Corso Italia, 31**
**I-34122 Trieste(IT)**

(72) Inventor: **Lovrecich, Mara Lucia**
**Via dei Moreri, 23**
**F-34100 Trieste(IT)**

(74) Representative: **Gervasi, Gemma et al**
**NOTARBARTOLO & GERVASI Srl Viale**
**Bianca Maria 33**
**I-20122 Milan (IT)**

Rank Xerox (UK) Business Services
(3.10/3.6/3.3.1)

EP 0 364 944 B1

**Description**

This invention relates to poorly soluble medicaments supported on polymer substances.

Prior art

In numerous cases, orally administered medicaments are poorly adsorbed with the result that their hematic concentration is low and varies between subjects. These problems are very often due to poor water-solubility and wettability of the medicament. Various treatments have been used to overcome these problems, such as micronization and addition of surfactants, but the improvement is often very limited.

To improve the dissolution of poorly soluble medicaments it has recently been proposed to support said medicaments in crosslinked polymers which are swellable but not soluble in water.

B. Lippold et al. (D.O.S. 26 34 004) have patented a process which consists of incorporating medicament solutions into said polymers and drying the product obtained. An increase in dissolving rate has been found for diphenylhydantoin, tolbutamide and griseofulvin.

D.O.S. patent 33 20 583 describes incorporating sulphonylurea derivatives in water-insoluble supports, such as crospovidone (crosslinked polyvinylpyrrolidone) using solutions containing water-soluble polymers such as linear polyvinylpyrrolidone. This process also results in an increase in the medicament dissolving rate.

EP patent application 78 430 relates to dihydropyridine derivatives adsorbed on crospovidone together with polyvinylpyrrolidone, these demonstrating very fast absorption. SA patent application 87/0738 describes incorporating dihydropyridine/crospovidone adsorbates in a water-gellable polymer or mixture of polymers, to obtain improved release and absorption.

A considerable increase in hematic level has been obtained by administering medroxyprogesterone acetate incorporated in crospovidone (G.B. patent application 840,3360). Other processes have also been proposed for incorporation into said water-insoluble polymers.

For example, G.B. patent 2,153,676 describes a co-heating method by which a mixture of an insoluble polymer and a medicament is heated to the medicament melting point with consequent incorporation of the molten medicament in the polymer lattice.

G.B. patent 2,153,678 relates to a co-grinding process by which a mixture of insoluble polymers and a medicament is ground in high-energy mill to produce improvement in the medicament dissolving characteristincs deriving from incorporating the medicament molecules in the polymer.

In addition a process is described (DE-A-3 326 167) in which a solution of glibenclamid in chloroform is incorporated into crosslinked polyvinylpyrrolidone and dried.

The powder obtained is treated with water at room temperature with the purpose of removing the organic solvent.

The water is removed and the residue is dried. A product with a low content of chloroform is obtained.

Summary

We have now found that the dissolving rate of poorly soluble medicaments can be considerably increased by a method characterised by:
1) incorporating the medicament into particles of a water-swellable but insoluble crosslinked polymer;
2) bringing the product obtained in stage 1) into contact with a solvent selected from the group consisting of methanol, ethanol, higher alcohols, acetone, chlorinated solvents, formamide, DMF and fluorinated hydrocarbons, in gaseous or liquid form, which is able to swell the polymer;
3) drying the product obtained in stage 2) under vacuum.

The invention relates to said method, the product obtained and pharmaceutical compositions which contain it.

The product obtained is characterised in that the polymer particles have a medicament concentration in their surface layers which is higher than that in their inner layers, the medicament being in the form of particles of nanometre dimensions.

Detailed description of the invention

The characteristics and advantages of the method and product according to the invention will be more apparent from the following detailed description.

The method according to the invention is implemented in two stages as follows:

2

1st stage:

In the 1st stage the medicament is incorporated into a water-swellable but water-insoluble crosslinked polymer (or mixture of two or more such polymers) by any known method such as any of the following:

1a) the medicament is dissolved in a suitable solvent and a certain volume of the solution is sprayed onto a given quantity of polymer with the weight ratio of solution to polymer being chosen on the basis of the polymer swelling capacity and on the basis of the concentration of the medicament in the solution.

The spraying can be carried out in any apparatus used for the purpose, such as in a continuously stirred reactor, in a rotary evaporator under continuous rotation, in a vacuum granulator under constant mixing, in a mortar under light mixing with a pestle, or in a fluidised bed with the polymer kept suspended in an air stream. The product obtained is then dried in the aforesaid apparatuses or in other suitable apparatuses.

1b) the medicament is dissolved in a suitable solvent and a quantity of a water-swellable but water-insoluble crosslinked polymer (or a mixture of two or more such polymers) is suspended in an excess of the solution obtained.

The suspension is kept stirred until the polymer particles swell. The suspension is then filtered or separated by other suitable means and the product is recovered and dried.

1c) the medicament in powder form and the water-swellable but water-insoluble crosslinked polymer (or mixture of two or more such polymers) in powder form are homogeneously mixed together and then ground together in a suitable apparatus such as a ball mill, high-anergy vibratory mill, air jet mill etc.

1d) the medicament in powder form and the water-swellable but water-insoluble crosslinked polymer in powder form are mixed homogeneously and then heated together to the medicament melting point in an apparatus such as an oven, rotary evaporator, reaction vessel, oil bath etc. until the medicament has melted and has been absorbed by the polymer.

The weight ratio of the medicament to said polymer (or mixture of two or more polymers) is in all cases between 0.1 and 1000 parts by weight of medicament per 100 parts by weight of polymer and preferably between 10 and 100 parts by weight of medicament per 100 parts by weight of polymer.

2nd stage:

In the 2nd stage the polymer in which the medicament has been incorporated by any of the methods described for the 1st stage is brought into contact with a solvent in the vapour or liquid state by any suitable method, for example by any of the following:

2a) the polymer with the medicament incorporated is introduced into a chamber into which the solvent in vapour form is fed through a valve. The chamber can be that in which the 1st stage was carried out;

2b) the polymer with the medicament incorporated is introduced into a sealed chamber already saturated with solvent vapour generated by a solvent container situated within the chamber and kept in the sealed chamber until saturation is complete;

2c) the polymer with the medicament incorporated is suspended in a fluidised bed by an air stream and is then sprayed with the liquid solvent or is exposed to an air stream saturated with the solvent vapour;

2d) the polymer with the medicament incorporated is suspended in an excess of solvent in liquid form, for example in a reaction vessel, in a mixer etc., and is then filtered off or separated by other means.

The time of contact between the polymer with the medicament incorporated and the solvent in vapour or liquid form is defined specifically for each medicament/polymer/solvent combination in order to obtain the desired characteristics of high medicament concentration in the surface layers and/or transformation of the physical state of the medicament into a crystalline state of lower melting point.

The treatment with solvent in gaseous form is conducted at a temperature preferably of between 20 and 100 °C and the treatment with solvent in liquid form is conducted at a temperature preferably of between 5 and 90 °C.

The time of contact with the gaseous solvent is between 0.5 and 48 hours. The time of contact with the liquid solvent is between 1 minute and 96 hours. The final drying of the product is preferably conducted in an oven under vacuum at a temperature of between 20 and 100 °C.

Examples of water-swellable but water-insoluble crosslinked polymers suitable for use (singly or in combinations of two or more than two) in the process of the invention are:

- crosslinked polyvinylpyrrolidone (abbreviated to crospovidone) as described in National Formulary XV, Supplement 3, page 368;
- crosslinked sodium carboxymethylcellulose as described in National Formulary XV, Supplement 3, page 367;

- crosslinked β-cyclodextrin polymer as described in WO patent 83/00809 and by Fenyvest et al. in Pharmacie, 39, 473, 1984;
- crosslinked dextran.

Of particular interest is the fact that the present invention has shown a new use for crosslinked β-cyclodextrin polymer, a substance which up to now has been used only as a disintegrator for solid pharmaceutical compositions and not as a support for medicaments.

However, according to the present invention, any polymer can be used which has the following characteristics:

- hydrophilic polymer lattice allowing high swellability in water;
- water insolubility deteròined by the nature of the polymer lattice.

The medicaments which can be used according to the present invention are preferably those which have limited water-solubility such as griseofulvin, indomethacin, diacerein, nicergoline, megestrol, progesterone, nifedipine, diltiazem, piroxicam, medroxyprogesterone acetate, clonidine, estradiol, etoposide, lorazepam and temazepam.

The non-aqueous solvent (or solvent mixtures) suitable for use in the method according to be invention are all those which are able to swell the polymer or to be absorbed by the polymer into which the medicament has been incorporated.

Examples of solvents are methanol, ethanol, higher alcohols, acetone, chlorinated solvents, formamide, DMF, fluorinated hydrocarbons.

The only limitation on the choice of solvent is that in the aforesaid case 2d), the solvent must have very limited dissolving power so that during treatment in the solvent, the quantity of medicament which is dissolved is limited.

One of the main characteristics of the product obtained by the method of the invention is that the particles have a medicament concentration in the surface layers which is higher than that of products obtained by the known art, i.e. the medicament is preferentially localized in the surface layers of the particles instead of in their interior. This results in a higher medicament release rate, as the diffusion of the medicament molecules is much less hindered by the internal polymer lattice.

The high surface concentration of the medicament in the product of the present invention is demonstrated by X-ray photoelectronic spectroscopic analysis (XPS) which allows quantitative elementary investigation of the outer layers of the polymer as far as 10 nm (100 Å) from the surface.

A description of the XPS method is given in Kane P.F. et al., Characterization of Solid Surface, Plenum Press, N.Y. 1978, pp 307-336.

This technique has been applied successfully to particles of crospovidone with incorporated medicament (F. Carli et al., J. Pharm. Sci., 963, 1985)

A hypothetical interpretation of the fact that a higher medicament concentration is obtained in the surface layers with the method of the invention is that this is determined mainly by the opening of the polymer lattice due to the solvent/polymer interaction when the polymer particles with incorporated medicament are treated with the solvent and also by the co-migration effect due to the solvent/medicament interaction during the final drying of the particles.

Another possible mechanism which can intervene in the preparation of the product according to the invention is that the physical state of the medicament can be transformed from the strongly metastable amorphous state to a stable crystalline state having a much higher energy level than the original crystalline state of the pure medicament, as shown by the melting point being much lower than that of the pure medicament.

This high-energy crystalline state results in a high dissolving rate as in the case of the amorphous state, but has a much higher storage life than this latter.

The lower melting point of the medicament incorporated in the polymer according to the present invention can be attributed to the formation of very small crystals, in the nanometre range, within the polymer lattice. A theoretical explanation of this depressed melting point, based on the thermodynamic treatment of three-phase equilibria in systems of large surface area, has recently been proposed by F. Carli et al., Proceedings of the 13th Controlled Release Bioactive Material Symp., Norfolk, USA, 1986; Proceedings of Ind. Pharm. Techn. Conference, London, 1988.

The reduction in melting point can be measured by DSC (differential scanning calorimetry) which also enables the residual crystallinity percentage to be determined by measuring the fusion energy in the sample.

The medicaments of this invention may be used for preparing capsules and tablets with controlled release, suspensions and transdermal films.

The following examples are given as non-limiting illustration of the invention.

4

EXAMPLE 1

1st stage:

10 g of crospovidone (Kollidon Cl, BASF) were swollen by slow addition of 20 ml of a 100 mg/ml solution of griseofulvin in dimethylformamide, mixing the powder continuously in a mortar.

The powder swollen in this manner was then placed in an oven under vacuum at a temperature of 100°C for 12 hours, until it was completely dried.

2nd stage:

2 g of the product obtained in the first stage were disintegrated through a sieve (14 mesh) and then placed in a hermetically sealed container at ambient temperature, saturated with methylenechloride vapour from a receptacle filled with this solvent and placed in the container. After 24 hours the powder treated in this manner was dried for 1 hour at 30°C in an oven under vacuum, sieved through a 14 mesh sieve and mixed for 10 minutes.

To allow a comparison between the Example 1 and a method using water as solvent in second stage, the Examples 2 and 3 were conducted.

EXAMPLE 2 (COMPARISON)

1st stage:

10 g of crospovidone (Knollidon Cl, BASF) were swollen by slow addition of 2 ml of a 100 mg/ml solution of griseofulvin in dimethylformamide, mixing the powder continuously in a mortar.

The powder swollen in this manner was then placed in an oven under vacuum at a temperature of 100°C for 12 hours, until it was completely dried.

2nd stage:

2 g of the powder with incorporated medicament obtained in the first stage were placed in a drier at ambient temperature and under an internal humidity of 90-92% obtained by an aqueous solution of suitable salts placed at the base of the same drier below the perforated floor on which the powder to be treated is placed. After 24 hours the powder treated in this manner was dried for 1 hour at 80°C in an oven under vacuum, sieved through a 14 mesh sieve and mixed for 10 minutes.

EXAMPLE 3 (COMPARISON)

1st stage:

10 g of crospovidone (Kollidon Cl, BASF) were swollen by slow addition of 20 ml of a 100 mg/ml solution of griseofulvin in dimethylformamide, mixing the powder continuously in a mortar.

The powder swollen in this manner was then placed in an oven under vacuum at a temperature of 100°C for 12 hours, until it was completely dried.

2nd stage:

1 g of the powder with incorporated medicament obtained in the first stage was wetted with 1 ml of demineralized water in a mortar, mixing the powder slowly for 1,5 hours. The swollen powder was dried for 1 hour at 80°C in an oven under vacuum.It was then disintegrated through a 14 mesh sieve and mixed for 10 minutes.

EXAMPLE 4

1st stage:

2.5 g of diacerein and 7.5 of crospovidone were sieved separately through a 14 mesh sieve and mixed for 10 minutes.

They were then introduced together with the grinding means into the grinding chamber of a high-energy vibration ball mill and ground for 1 hour. They were then disintegrated through a 14 mesh sieve.

2nd stage:

8 g of the crospovidone powder with diacerein incorporated as in stage 1 were introduced into a hermetically sealed container saturated with methylenechloride vapour from a receptacle situated in said container and containing said solvent. After exposure to the vapour for 24 hours at ambient temperature the powder was dried in an oven under vacuum at ambient temperature for 2 hours and then disintegrated through a 14 mesh sieve and mixed.

EXAMPLE 5

1st stage:

1 g of crospovidone (Kollidon Cl, BASF) was swollen by slow addition of 1.7 ml of a 196 mg/ml solution of megestrol acetate in methylenechloride while continuously mixing the powder in a mortar. The powder swollen in this manner was then left in an oven under vacuum at ambient temperature for two hours until it was completely dry.

2nd stage:

The powder obtained in the 1st stage was disintegrated through a sieve (No. 14 mesh) and then placed in a hermetically sealed container at ambient temperature, which was saturated with methylenechloride vapour from a receptacle containing said solvent and placed in the container. After 24 hours the treated powder was dried for 1 hour at 30°C in an oven under vacuum, sieved through a 14 mesh sieve and mixed for 10 minutes.

EXAMPLE 6

1st stage:

0.95 g of crospovidone (Kollidon CL-M, BASF) were swollen by 2 ml a 95 mg/ml solution of nifedipine in methylenechloride while continuously mixing in a mortar. The swollen powder was then dried in an oven under vacuum at 30°C for 3 hours until it was completely dry.

2nd stage:

The powder obtained in the 1st stage was sieved through a 14 mesh sieve and mixed and then placed in a hermetically sealed container at 25°C which was saturated with methylenechloride vapour at a vapour pressure corresponding to the solvent temperature of 25°C.

After 24 hours the treated powder was dried at 30°C for 3 hours in an oven under vacuum, sieved through a 14 mesh sieve and mixed for 10 minutes.

EXAMPLE 7

1st stage:

142 g of crospovidone (Kollidon CL-M, BASF) were swollen by 300 ml of a 95 mg/ml solution of nifedipine in methylenechloride; this solution was added to the crospovidone powder in the mixing chamber of a high speed granulator. The swollen powder was then dried in the same mixer at 30°C and 400 mbar for 2 hours.

2nd stage:

The powder obtained in the 1st stage was further swollen in the same mixer with 250 ml of methylenechloride at 25°C while mixing.

At the end of the addition the mass was mixed for 24 hours in an environment saturated with the solvent. Subsequently the mass was dried at 30°C and 400 mbar for 2 hours in the same mixer.

EXAMPLE 8

1st stage:

2 g of crospovidone (Kollidon CL-M, BASF) were swollen by 4 ml of a 20 mg/ml solution of nicergoline in methylenechloride while continuously mixing in a mortar. The swollen powder was then dried in an oven under vacuum at 30°C for 3 hours until it was completely dry.

2nd stage:

The powder obtained in the 1st stage was sieved through a 14 mesh sieve and mixed and then placed in a hermetically sealed container at 25°C which was saturated with methylenechloride vapour at a vapour pressure corresponding to the solvent temperature of 25°C.

After 24 hours the treated powder was dried at 30°C for 3 hours in an oven under vacuum, sieved through a 14 mesh sieve and mixed for 10 minutes.

To allow a comparison between the aforesaid examples implemented in accordance with the method of the present invention and the methods of the known art, the following examples 9 to 11 were conducted using known methods.

EXAMPLE 9 (comparison)

10 g of crospovidone were swollen with 20 ml of 100 mg/ml solution of griseofulvin in dimethylformamide, said solution being added slowly to the crospovidone powder which was kept under mixing in a mortar. The swollen powder was then dried in an oven under vacuum at a temperature of 100°C for 12 hours and then disintegrated through a 14 mesh sieve and mixed for 10 minutes.

EXAMPLE 10 (comparison)

2.5 of diacerein and 7.5 g of crospovidone were sieved through a 14 mesh sieve and then mixed for 10 minutes. The mixture was then introduced together with the grinding means into the grinding chamber of a high-energy vibration ball mill and ground for 1 hour. The powder obtained was then disintegrated through a 14 mesh sieve and mixed for 10 minutes.

EXAMPLE 11 (comparison)

1 g of crospovidone (Kollidon Cl, BASF) was placed in a mortar and swollen by slow addition of 1.7 ml of a 196 mg/ml solution of megestrol acetate in methylenechloride while continuously mixing. The swollen powder was then dried in an oven under vacuum at ambient temperature for 2 hours until it was completely dried and then disintegrated through a 14 mesh sieve and mixed for 10 minutes.

Determination of the dissolving rate

The dissolving rate data for the products prepared by the method of this invention (Examples 1, 4, 5, 6, 7 and 8) are given in Tables 1-4.

For comparison, said table also give the dissolving rate data for the products prepared by known methods (Examples 2, 3, 9, 10 and 11).

For all the medicaments studied, the method used was the U.S.P. XX No. 2 method using a SOTAX apparatus at 37°C and a Beckman Du 65 spectrophotometer.

For the products containing griseofulvin 900 ml of a pH 7.5 buffer solution were used while stirring at 150 r.p.m. The spectrophotometric reading of the suitably diluted samples was taken at 294 nm.

For the products containing diacerein, 900 ml of a pH 5.5 buffer solution were used while stirring at 100 r.p.m.The spectrophotometric reading was taken at 255 nm.

For the products containing nifedipine, 900 ml of a pH 7.5 buffer solution were used while stirring at 150 r.p.m. The spectrophotometric reading was taken at 330 nm.

For the products containing megestrol acetate, 900 ml of a pH 5.2 buffer solution were used while stirring at 150 r.p.m.

For the determination of concentration, an HPLC of SPECTRA PHYSICS Mod. SP4A290/SP8800 was used, with mobile phase acetonitrile-water 50/50 v/v and flow rate 1 ml/min with a NOVAPAK $C_{18}$ colum and Mod. SP8490 UV detector, Imoz 292 nm.

As can be observed from the data of Tables 1-4, in the case of all the medicaments and swellable polymers used, the dissolving rate was clearly greater for the products prepared by the method of the invention than for the analogous products prepared by the previously known methods.

TABLE 1

| Dissolving rate of products consisting of griseofulvin/crospovidone 1:5 w/w | | | | |
|---|---|---|---|---|
| Time | Griseofulvin concentration ($\mu$g/ml) | | | |
| | Comparison preparation (Example 9) | Preparation of invention (Example 1) | COMPARISON PREPARATION (Example 2) | COMPARISON PREPARATION (Example 3) |
| 5 min | 1.29 | 1.81 | 1.76 | 1.70 |
| 10 min | 1.53 | 2.55 | 2.25 | 2.67 |
| 15 min | 1.96 | 2.91 | 2.58 | 2.98 |
| 20 min | 2.41 | 3.42 | 2.86 | 3.31 |
| 30 min | 3.08 | 3.89 | 3.20 | 3.71 |
| 40 min | 3.38 | 4.24 | 3.85 | 3.85 |
| 60 min | 3.52 | 4.59 | 3.81 | 4.05 |
| N.B. The values reported are the mean of at least three repeats; maximum C.V. $\leq$ 8% | | | | |

TABLE 2

| Dissolving rate of products consisting of diacerein/crospovidone 1:3 w/w. | | |
|---|---|---|
| Time | Diacerein concentration ($\mu$g/ml) | |
| | Comparison preparation (Example 10) | Preparation of invention (Example 4) |
| 1 min | 9.78 | 9.08 |
| 3 min | 13.64 | 16.55 |
| 5 min | 15.27 | 19.86 |
| 10 min | 17.08 | 23.00 |
| 15 min | 18.26 | 24.97 |
| 30 min | 19.47 | 26.26 |
| 45 min | 21.95 | 26.25 |
| 60 min | 23.01 | 26.30 |

TABLE 3

| Dissolving rate of products consisting of megestrol acetate/crospovidone 1:3 w/w. | | |
|---|---|---|
| Time | Megestrol acetate concentration ($\mu$g/ml) | |
| | Comparison preparation (EXAMPLE 11) | Preparation of invention (Example 5) |
| 10 min | 0.0239 | 0.0397 |
| 20 min | 0.0438 | 0.0628 |
| 30 min | 0.0714 | 0.0934 |
| 45 min | 0.0891 | 0.1160 |
| 60 min | 0.1091 | 0.1382 |
| 90 min | 0.1610 | 0.1766 |
| 120 min | 0.1838 | 0.1927 |

TABLE 4

| Dissolving rate of products consisting of nifedipine/crospovidone 1:5 w/w. | | |
|---|---|---|
| Time | Nifedipine concentration ($\mu$g/ml). | |
| | Preparation of invention (Example 6) | Preparation of invention (Example 7) |
| 5 min | 3.71 | 4.03 |
| 10 min | 4.22 | 4.39 |
| 15 min | 4.46 | 4.23 |
| 30 min | 4.69 | 4.47 |
| 60 min | 4.82 | 4.73 |
| 90 min | 5.03 | 4.91 |

Differential scanning calorimetry data

A further characteristic of the products prepared by the method of the present invention is that they have a high-energy crystalline state, ie they have a melting point which is lower than that of the medicament as such. This lowering of melting point is due to the formation of very fine crystals in the nanometre dimensional range (so-called nanocrystals).

Table 5 shows the thermoanalytical data relative to products prepared according to the invention, the data being obtained using a differential scanning calorimeter TA 3000 of Mettler (Switzerland) with nitrogen flow and a heating rate of 10°K min$^{-1}$. For comparison purposes Table 5 also shows data relative to products prepared by the conventional method, whereas Table 6 shows the thermoanalytical characteristics of the crystalline active principles as such.

Comparing the data for products prepared in accordance with the invention with those for the products prepared by conventional methods and those of the active principles as such, it can be seen that in every case the activation by treatment with solvent leads to polymer products containing incorporated medicament crystals having melting point clearly less than that of the medicaments as such. In contrast, the conventional preparation method produces products having the same melting point as the medicaments as such.

In this respect, for the griseofulvin/crospovidone products there is a melting point reduction of 13-30°C; for the nifedipine/crospovidone products there is a reduction of the order of 20°C; and for the diacerein/crospovidone products there is a melting point reduction of 40-50°C.

As stated, these melting point reductions indicate that the medicaments are in a crystalline state of higher energy than that of the medicaments as such, with consequent solubility increase. In addition, it has also already been stated that these melting point reductions are due to extremely fine crystal dimensions of nanometre level. Consequently the products activated by the method of the invention have improved bioavailability characteristics compared with the medicaments as such and with the products prepared by the conventional method.

TABLE 5 - Differential scanning calorimetry data for the medicament/swellable insoluble polymer products prepared by the method of the invention and by the traditional method.

|  | THERMAL CHARACTERISTICS | MELTING POINT | HEAT OF FUSION |
|---|---|---|---|
| Griseofulvin/crospovidone systems 1/5 w/w | | | |
| Example 9[a] | 1 PEAK ONLY | 219.0°C | 7.8 J/g |
| Example 1[b] | 1 PEAK ONLY | 194.2°C | 44.2 J/g |
| Diacerein/crospovidone systems 1/3 w/w: | | | |
| Example 10[a] | NO PEAK | -- | -- |
| Example 4[b] | 2 PEAKS | 176.0°C | 25.7 J/g |
|  |  | 202.7°C | 14.4 J/g |

a) product prepared by the traditional method

b) product prepared by the method of the invention

TABLE 6

| Differential scanning calorimetry data for active principles as such. | | | |
|---|---|---|---|
| ACTIVE PRINCIPLE | THERMAL CHARACTERISTICS | MELTING POINT | HEAT OF FUSION |
| Griseofulvin | 1 PEAK ONLY | 219.8°C | 120.4 J/g |
| Diacerein | 1 PEAK ONLY | 251.3°C | 229.3 J/g |

**Claims**

**Claims for the following Contracting States : AT, BE, CH, DE, FR, GB, IT, LI, LU, NL, SE**

1. A method for preparing medicaments supported on polymer substances in a form to increase the dissolving rate, characterised by:

   1) incorporating the medicament into particles of a crosslinked polymer which is swellable in water but is insoluble in water;

   2) bringing the product obtained in stage 1) into contact with a solvent selected from the group consisting of methanol, ethanol, higher alcohols, acetone, chlorinated solvents, formamide, DMF and fluorinated hydrocarbons, in gaseous or liquid form, which is able to swell the polymer;

   3) drying under vacuum the product obtained in stage 2).

2. A method as claimed in claim 1, characterised in that said contact with the solvent is effected in a chamber into which the solvent is fed in the form of vapour.

3. A method as claimed in claim 1, characterised in that said contact with the solvent is effected in a chamber saturated with solvent vapour generated within the chamber itself.

4. A method as claimed in claim 1, characterised in that said contact with the solvent is effected in a fluidised bed by spraying the particles with the liquid solvent.

5. A method as claimed in claim 1, characterised in that said contact with the solvent is effected in a fluidised bed by an air stream saturated with the solvent vapour.

6. A method as claimed in claim 1, characterised in that said contact with the solvent is effected by suspending the product in the solvent in liquid form.

7. A method as claimed in claim 1, characterised in that said contact with the solvent in gaseous form is conducted for a time of between 0.5 and 48 hours.

8. A method as claimed in claim 1, characterised in that said contact with the solvent in liquid form is conducted for a time of between 1 minute and 96 hours.

9. A method as claimed in claim 1, characterised in that said contact with the solvent in gaseous form is effected at a temperature of between 20 and 100°C.

10. A method as claimed in claim 1, characterised in that said contact with the solvent in liquid form is effected at a temperature of between 5 and 90°C.

11. A method as claimed in claim 1, characterised in that said drying is effected at a temperature of between 20 and 100°C.

12. A method as claimed in claim 1, characterised in that said polymer is crosslinked polyvinylpyrrolidone, crosslinked sodium carboxymethylcellulose, crosslinked $\beta$-cyclodextrin polymer or crosslinked dextran.

13. A method as claimed in claim 1, characterised in that said medicament is griseofulvin, indomethacin, diacerein, megestrol or nicergoline.

14. Poorly soluble medicaments supported on polymer substances in a form able to increase the dissolving rate, obtainable by the process claimed in claims 1 to 13.

15. The use of medicaments claimed in claim 14 for preparing capsules and tablets with controlled release, suspensions and transdermal films.

**Claims for the following Contracting States : GR, ES**

1. A method for preparing medicaments supported on polymer substances in a form to increase the dissolving rate, characterised by:
1) incorporating the medicament into particles of a crosslinked polymer which is swellable in water but is insoluble in water;
2) bringing the product obtained in stage 1) into contact with a solvent selected from the group consisting of methanol, ethanol, higher alcohols, acetone, chlorinated solvents, formamide, DMF and fluorinated hydrocarbons, in gaseous or liquid form, which is able to swell the polymer;
3) drying under vacuum the product obtained in stage 2).

2. A method as claimed in claim 1, characterised in that said contact with the solvent is effected in a chamber into which the solvent is fed in the form of vapour.

3. A method as claimed in claim 1, characterised in that said contact with the solvent is effected in a chamber saturated with solvent vapour generated within the chamber itself.

4. A method as claimed in claim 1, characterised in that said contact with the solvent is effected in a fluidised bed by spraying the particles with the liquid solvent.

**5.** A method as claimed in claim 1, characterised in that said contact with the solvent is effected in a fluidised bed by an air stream saturated with the solvent vapour.

**6.** A method as claimed in claim 1, characterised in that said contact with the solvent is effected by suspending the product in the solvent in liquid form.

**7.** A method as claimed in claim 1, characterised in that said contact with the solvent in gaseous form is conducted for a time of between 0.5 and 48 hours.

**8.** A method as claimed in claim 1, characterised in that said contact with the solvent in liquid form is conducted for a time of between 1 minute and 96 hours.

**9.** A method as claimed in claim 1, characterised in that said contact with the solvent in gaseous form is effected at a temperature of between 20 and 100°C.

**10.** A method as claimed in claim 1, characterised in that said contact with the solvent in liquid form is effected at a temperature of between 5 and 90°C.

**11.** A method as claimed in claim 1, characterised in that said drying is effected at a temperature of between 20 and 100°C.

**12.** A method as claimed in claim 1, characterised in that said polymer is crosslinked polyvinylpyrrolidone, crosslinked sodium carboxymethylcellulose, crosslinked $\beta$-cyclodextrin polymer or crosslinked dextran.

**13.** A method as claimed in claim 1, characterised in that said medicament is griseofulvin, indomethacin, diacerein, megestrol or nicergoline.

**Patentansprüche**

**Patentansprüche für folgende Vertragsstaaten : AT, BE, CH, DE, FR, GB, IT, LI, LU, NL, SE**

**1.** Verfahren zur Herstellung von Medikamenten (Arzneistoffen), die auf Polymersubstanzen als Träger aufgebracht sind, zur Erhöhung der Auflösungsrate, gekennzeichnet durch die folgenden Stufen:
1) Einarbeitung des Medikaments (Arzneistoffes) in die Teilchen eines vernetzten Polymers, das in Wasser quellbar, jedoch in Wasser unlöslich ist;
2) Inkontaktbringen des in der Stufe (1) erhaltenen Produkts mit einem Lösungsmittel, ausgewählt aus der Gruppe, die besteht aus Methanol, Ethanol, höheren Alkoholen, Aceton, chlorierten Lösungsmitteln, Formamid, DMF und fluorierten Kohlenwasserstoffen, in gasförmiger oder flüssiger Form, das in der Lage ist, das Polymer zum Aufquellen zu bringen;
3) Trocknen des in der Stufe (2) erhaltenen Produkts unter Vakuum.

**2.** Verfahren nach Anspruch 1, dadurch gekennzeichnet, daß der Kontakt mit dem Lösungsmittel in einer Kammer erfolgt, in welche das Lösungsmittel in Form eines Dampfes (Gases) eingeführt wird.

**3.** Verfahren nach Anspruch 1, dadurch gekennzeichnet, daß der Kontakt mit dem Lösungsmittel in einer Kammer erfolgt, die mit innerhalb der Kammer selbst erzeugtem Lösungsmitteldampf gesättigt ist.

**4.** Verfahren nach Anspruch 1, dadurch gekennzeichnet, daß der Kontakt mit dem Lösungsmittel in einem Wirbelbett erfolgt durch Besprühen der Teilchen mit dem flüssigen Lösungsmittel.

**5.** Verfahren nach Anspruch 1, dadurch gekennzeichnet, daß der Kontakt mit dem Lösungsmittel in einem Wirbelbett erfolgt mittels eines Luftstroms, der mit dem Lösungsmitteldampf gesättigt ist.

**6.** Verfahren nach Anspruch 1, dadurch gekennzeichnet, daß der Kontakt mit dem Lösungsmittel erfolgt durch Suspendieren des Produkts in dem Lösungsmittel in flüssiger Form.

**7.** Verfahren nach Anspruch 1, dadurch gekennzeichnet, daß der Kontakt mit dem Lösungsmittel in gasförmiger Form für eine Zeitspanne zwischen 0,5 und 48 h durchgeführt wird.

**8.** Verfahren nach Anspruch 1, dadurch gekennzeichnet, daß der Kontakt mit dem Lösungsmittel in flüssiger Form für eine Zeitspanne zwischen 1 min und 96 h durchgeführt wird.

**9.** Verfahren nach Anspruch 1, dadurch gekennzeichnet, daß der Kontakt mit dem Lösungsmittel in gasförmiger Form bei einer Temperatur zwischen 20 und 100°C erfolgt.

**10.** Verfahren nach Anspruch 1, dadurch gekennzeichnet, daß der Kontakt mit dem Lösungsmittel in flüssiger Form bei einer Temperatur zwischen 5 und 90°C erfolgt.

**11.** Verfahren nach Anspruch 1, dadurch gekennzeichnet, daß das Trocknen bei einer Temperatur zwischen 20 und 100°C duchgeführt wird.

**12.** Verfahren nach Anspruch 1, dadurch gekennzeichnet, daß das Polymer vernetztes Polyvinylpyrrolidon, vernetzte Natriumcarboxymethylcellulose, vernetztes β-Cyclodextrin-Polymer oder vernetztes Dextran ist.

**13.** Verfahren nach Anspruch 1, dadurch gekennzeichnet, daß das Medikament (der Arzneistoff) Griseofulvin, Indomethacin, Diacerein, Megestrol oder Nicergolin ist.

**14.** Schwerlösliche Medikamente (Arzneistoffe), die auf Polymersubstanzen als Träger aufgebracht sind in einer Form, die eine Erhöhung der Auflösungsrate ermöglicht, wie sie nach dem Verfahren gemäß den Ansprüchen 1 bis 13 erhältlich sind.

**15.** Verwendung der Medikamente (Arzneistoffe) nach Anspruch 14 zur Herstellung von Kapseln und Tabletten mit kontrollierter Freisetzung, von Suspensionen und transdermalen Filmen.

**Patentansprüche für folgende Vertragsstaaten : GR, ES**

**1.** Verfahren zur Herstellung von Medikamenten (Arzneistoffen), die auf Polymersubstanzen als Träger aufgebracht sind, zur Erhöhung der Auflösungsrate, gekennzeichnet durch die folgenden Stufen:
1) Einarbeitung des Medikaments (Arzneistoffes) in die Teilchen eines vernetzten Polymers, das in Wasser quellbar, jedoch in Wasser unlöslich ist;
2) Inkontaktbringen des in der Stufe (1) erhaltenen Produkts mit einem Lösungsmittel, ausgewählt aus der Gruppe, die besteht aus Methanol, Ethanol, höheren Alkoholen, Aceton, chlorierten Lösungsmitteln, Formamid, DMF und fluorierten Kohlenwasserstoffen, in gasförmiger oder flüssiger Form, das in der Lage ist, das Polymer zum Aufquellen zu bringen;
3) Trocknen des in der Stufe (2) erhaltenen Produkts unter Vakuum.

**2.** Verfahren nach Anspruch 1, dadurch gekennzeichnet, daß der Kontakt mit dem Lösungsmittel in einer Kammer erfolgt, in welche das Lösungsmittel in Form eines Dampfes (Gases) eingeführt wird.

**3.** Verfahren nach Anspruch 1, dadurch gekennzeichnet, daß der Kontakt mit dem Lösungsmittel in einer Kammer erfolgt, die mit innerhalb der Kammer selbst erzeugtem Lösungsmitteldampf gesättigt ist.

**4.** Verfahren nach Anspruch 1, dadurch gekennzeichnet, daß der Kontakt mit dem Lösungsmittel in einem Wirbelbett erfolgt durch Besprühen der Teilchen mit dem flüssigen Lösungsmittel.

**5.** Verfahren nach Anspruch 1, dadurch gekennzeichnet, daß der Kontakt mit dem Lösungsmittel in einem Wirbelbett erfolgt mittels eines Luftstroms, der mit dem Lösungsmitteldampf gesättigt ist.

**6.** Verfahren nach Anspruch 1, dadurch gekennzeichnet, daß der Kontakt mit dem Lösungsmittel erfolgt durch Suspendieren des Produkts in dem Lösungsmittel in flüssiger Form.

**7.** Verfahren nach Anspruch 1, dadurch gekennzeichnet, daß der Kontakt mit dem Lösungsmittel in gasförmiger Form für eine Zeitspanne zwischen 0,5 und 48 h durchgeführt wird.

**8.** Verfahren nach Anspruch 1, dadurch gekennzeichnet, daß der Kontakt mit dem Lösungsmittel in flüssiger Form für eine Zeitspanne zwischen 1 min und 96 h durchgeführt wird.

**9.** Verfahren nach Anspruch 1, dadurch gekennzeichnet, daß der Kontakt mit dem Lösungsmittel in gasförmiger Form bei einer Temperatur zwischen 20 und 100°C erfolgt.

**10.** Verfahren nach Anspruch 1, dadurch gekennzeichnet, daß der Kontakt mit dem Lösungsmittel in flüssiger Form bei einer Temperatur zwischen 5 und 90°C erfolgt.

**11.** Verfahren nach Anspruch 1, dadurch gekennzeichnet, daß das Trocknen bei einer Temperatur zwischen 20 und 100°C duchgeführt wird.

**12.** Verfahren nach Anspruch 1, dadurch gekennzeichnet, daß das Polymer vernetztes Polyvinylpyrrolidon, vernetzte Natriumcarboxymethylcellulose, vernetztes β-Cyclodextrin-Polymer oder vernetztes Dextran ist.

**13.** Verfahren nach Anspruch 1, dadurch gekennzeichnet, daß das Medikament (der Arzneistoff) Griseofulvin, Indomethacin, Diacerein, Megestrol oder Nicergolin ist.

**Revendications**
**Revendications pour les Etats contractants suivants : AT, BE, CH, DE, FR, GB, IT, LI, LU, NL, SE**

**1.** Procédé de préparation de médicaments à base de substances polymères dans une forme permettant d'augmenter le taux de dissolution, caractérisé par :
1) l'incorporation du médicament dans des particules d'un polymère réticulé qui se gonfle dans l'eau mais y est insoluble;
2) la mise en contact du produit obtenu au stade 1) avec un solvant choisi parmi un groupe composé de méthanol, d'éthanol, d'alcools plus élevés, d'acétone, de solvants chlorés, de formamide, de diméthylformamide et d'hydrocarbones fluorés, sous forme gazeuse ou liquide, capable de faire gonfler le polymère;
3) mise à sec sous vide du produit obtenu au stade 2);

**2.** Procédé selon la revendication 1, caractérisé en ce que le contact avec le solvant est effectué dans une chambre dans laquelle le solvant est injecté sous forme de vapeur.

**3.** Procédé selon la revendication 1, caractérisé en ce que le contact avec le solvant est effectué dans une chambre saturée avec de la vapeur de solvant produite à l'intérieur de la chambre elle-même.

**4.** Procédé selon la revendication 1, caractérisé en ce que le contact avec le solvant est effectué sur un lit fluidisé par une projection de particules du liquide solvant.

**5.** Procédé selon la revendication 1, caractérisé en ce que le contact avec le solvant est effectué sur un lit fluidisé par un courant d'air saturé de vapeur du solvant.

**6.** Procédé selon la revendication 1, caractérisé en ce que le contact avec le solvant est effectué en mettant le produit en suspension dans le solvant sous une forme liquide.

**7.** Procédé selon la revendication 1, caractérisé en ce que le contact avec le solvant sous forme gazeuse est réalisé pendant une période de temps comprise entre 0,5 et 48 heures.

**8.** Procédé selon la revendication 1, caractérisé en ce que le contact avec le solvant sous forme liquide est réalisé pendant une période de temps comprise entre 1 minute et 96 heures.

**9.** Procédé selon la revendication 1, caractérisé en ce que le contact avec le solvant sous forme gazeuse est effectué à une température comprise entre 20 et 100° C.

**10.** Procédé selon la revendication 1, caractérisé en ce que le contact avec le solvant sous forme liquide est effectué à une température comprise entre 5 et 90° C.

**11.** Procédé selon la revendication 1, caractérisé en ce que le séchage est effectué à une température comprise entre 20 et 100° C.

**12.** Procédé selon la revendication 1, caractérisé en ce que le polymère est une polyvinylpyrrolidone réticulée, une carboxyméthylcellulose de sodium réticulée, un polymère de *β*-cyclodextrine réticulée ou du dextran réticulé.

**13.** Procédé selon la revendication 1, caractérisée en ce que le médicament est de la griséofulvine, de l'indométacine, de la diacéréine, de la mégestrole ou de la nicergoline.

**14.** Médicaments médiocrement solubles soutenus par des substances polymères sous une forme susceptible d'augmenter le taux de dissolution, obtenue par le procédé décrit dans les revendications 1 à 13.

**15.** Utilisation de médicaments selon la revendication 14 pour préparer des gélules ou comprimés à effet retard, des solutions et des films transdermiques.

**Revendications pour les Etats contractants suivants : GR, ES**

**1.** Procédé de préparation de médicaments à base de substances polymères dans une forme permettant d'augmenter le taux de dissolution, caractérisé par :
  1) l'incorporation du médicament dans des particules d'un polymère réticulé qui se gonfle dans l'eau mais y est insoluble;
  2) la mise en contact du produit obtenu au stade 1) avec un solvant choisi parmi un groupe composé de méthanol, d'éthanol, d'alcools plus élevés, d'acétone, de solvants chlorés, de formamide, de diméthylformamide et d'hydrocarbones fluorés, sous forme gazeuse ou liquide, capable de faire gonfler le polymère;
  3) mise à sec sous vide du produit obtenu au stade 2);

**2.** Procédé selon la revendication 1, caractérisé en ce que le contact avec le solvant est effectué dans une chambre dans laquelle le solvant est injecté sous forme de vapeur.

**3.** Procédé selon la revendication 1, caractérisé en ce que le contact avec le solvant est effectué dans une chambre saturée avec de la vapeur de solvant produite à l'intérieur de la chambre elle-même.

**4.** Procédé selon la revendication 1, caractérisé en ce que le contact avec le solvant est effectué sur un lit fluidisé par une projection de particules du liquide solvant.

**5.** Procédé selon la revendication 1, caractérisé en ce que le contact avec le solvant est effectué sur un lit fluidisé par un courant d'air saturé de vapeur du solvant.

**6.** Procédé selon la revendication 1, caractérisé en ce que le contact avec le solvant est effectué en mettant le produit en suspension dans le solvant sous une forme liquide.

**7.** Procédé selon la revendication 1, caractérisé en ce que le contact avec le solvant sous forme gazeuse est réalisé pendant une période de temps comprise entre 0,5 et 48 heures.

**8.** Procédé selon la revendication 1, caractérisé en ce que le contact avec le solvant sous forme liquide est réalisé pendant une période de temps comprise entre 1 minute et 96 heures.

**9.** Procédé selon la revendication 1, caractérisé en ce que le contact avec le solvant sous forme gazeuse est effectué à une température comprise entre 20 et 100 ° C.

**10.** Procédé selon la revendication 1, caractérisé en ce que le contact avec le solvant sous forme liquide est effectué à une température comprise entre 5 et 90 ° C.

**11.** Procédé selon la revendication 1, caractérisé en ce que le séchage est effectué à une température comprise entre 20 et 100 ° C.

**12.** Procédé selon la revendication 1, caractérisé en ce que le polymère est une polyvinylpyrrolidone réticulée, une carboxyméthylcellulose de sodium réticulée, un polymère de *β*-cyclodextrine réticulée ou du dextran réticulé.

15

13. Procédé selon la revendication 1, caractérisée en ce que le médicament est de la griséofulvine, de l'indométacine, de la diacéréine, de la mégestrole ou de la nicergoline.